(19) Europäisches Patentamt / European Patent Office / Office européen des brevets

(11) **EP 1 778 347 B1**

(12) **EUROPEAN PATENT SPECIFICATION**

(45) Date of publication and mention of the grant of the patent:
**13.04.2016 Bulletin 2016/15**

(21) Application number: **05772643.2**

(22) Date of filing: **07.07.2005**

(51) Int Cl.:
*A61N 1/39* (2006.01)     *A61B 5/053* (2006.01)

(86) International application number:
**PCT/EP2005/007454**

(87) International publication number:
**WO 2006/005557 (19.01.2006 Gazette 2006/03)**

(54) **DEFIBRILLATOR WITH CARDIAC BLOOD FLOW DETERMINATION**

DEFIBRILLATOR MIT BESTIMMUNG DES KARDIALEN BLUTFLUSSES

PROCEDE SERVANT A DETERMINER LA CIRCULATION SANGUINE CARDIAQUE ET DEFIBRILLATEUR METTANT EN APPLICATION CE PROCEDE

(84) Designated Contracting States:
**AT BE BG CH CY CZ DE DK EE ES FI FR GB GR HU IE IS IT LI LT LU LV MC NL PL PT RO SE SI SK TR**

(30) Priority: **09.07.2004 IE 20040468**

(43) Date of publication of application:
**02.05.2007 Bulletin 2007/18**

(73) Proprietor: **Heartsine Technologies Limited Belfast BT3 9ED (GB)**

(72) Inventors:
• **ALLEN, James,
Sycamore Stables
County Antrim BT39 OBS (GB)**

• **ANDERSON, John, McCune
Holywood, County Down BT18 0NG (GB)**

(74) Representative: **Armstrong, Rosemary et al
Armstrong IPR Limited
Innovation Centre
NI Science Park
Queens Road
Belfast, BT3 9DT (GB)**

(56) References cited:
**WO-A-03/022143**     **US-A- 4 619 265**
**US-A1- 2003 109 790**

**Description**

This invention relates to a method of determining whether events in a heart's electrical activity result in corresponding blood flow events, and to an external defibrillator implementing such method.

Out of hospital survival from sudden cardiac death (SCD) is now a regular occurrence[1]. The most important factor influencing survival is the time from the onset of the condition to treatment being administered. Since automatic external defibrillators (AEDs) allow treatment by emergency services and trained personnel on site rather than requiring that the patient is transported to a medical facility, their use is becoming more widespread on a daily basis. More recently their use is being made a requirement on flights, in some public places and offices by government bodies. There is also clinical and public pressure to make them available to family members and smaller institutions.

A modem AED is a compact portable unit which automatically analyses electrocardiograph (ECG) rhythms obtained from the human thorax via electrodes in contact with the skin. A user attempting to respond to a suspected SCD emergency would simply get the AED and press the "ON" button and then follow the voice and text prompts and instructions generated by the device. A typical sequence of events would be for the device to prompt the user to apply the pads to the patients chest and make sure that the electrodes are properly connected to the device. Once the device is satisfied that this has been performed, the device will then automatically analyse the patients heart rhythm and make a diagnosis. If it determines that a high voltage shock is required, it will automatically charge to a predetermined energy level and advise the user to press a clearly marked "SHOCK" button. Upon pressing this button, the energy is delivered to the patient through the same electrodes used to monitor the ECG. Whether or not the device advises shock, the user is informed continuously about the state of the patient and/or the actions and states of the device.

In order to analyse the patients ECG these AED's use an algorithm which employs various parameters measured from the digitised ECG[2-3]. Typically these parameters include frequency and amplitude of the ECG rhythm and also some integration techniques such as slope, morphology and heart rate. Many algorithms also utilise zero content or baseline content and energy ratio calculations. Calculating the mathematical variance of some parameters can also improve the performance of these devices.

All of the AED's using this approach however suffer from the fact that they are analysing the ECG or parameters measured thereof. Although there is a great deal of literature to show how the ECG is related to the mechanical and haemodynamic response of the heart, there are many instances where ECG activity is present when there is very little or no cardiac output. Furthermore many heart rhythms can exhibit a random and fluctuating nature while maintaining a sufficient cardiac output. The algorithms therefore can and do advise and fail to advise incorrectly[4-6]. What is really required is a device which can correctly identify and differentiate a cardiac rhythm associated with no pulse or haemodynamic collapse from those with satisfactory cardiac output.

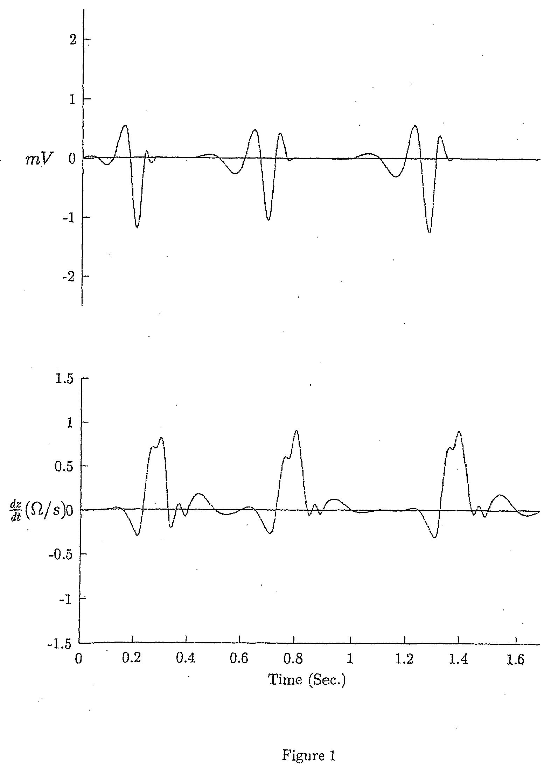

More recent work in the field of impedance cardiography[7] has enabled an impedance cardiograph (ICG) to be measured from the same two electrodes used to acquire the ECG and deliver the energy to the patient. Historically measuring the ICG had required four band type electrodes in order to achieve satisfactory accuracy. Utilising the electrodes already employed for ECG allows the ICG to be easily measured as another signal and source of information. The ICG contains information relating to the presence of cardiac output rather than electrical cardiac activity and can therefore be used to help classify heart rhythms more accurately and consistently. Referring now to the top trace in Figure 1, we see an example ECG of a subject in normal sinus rhythm (SR). The bottom trace shows the corresponding time synchronised trace of that same individuals ICG differentiated with respect to (wrt) time, dz/dt. This dz/dt signal is more commonly used for analysis rather than the raw ICG itself and so for the rest of this document the term ICG will be used to actually denote the signal dz/dt. Where the raw ICG sensed from the patient electrodes is referenced it will be explicitly named as such. For reference the first derivative of the raw ICG signal is derived as follows:

$$\mathrm{d}Z/\mathrm{d}t = \mathrm{d}[Z - Z_o]/\mathrm{d}t \qquad (1)$$

where Z is the raw impedance measured from the patients thorax and $Z_o$ is the baseline impedance of Z.

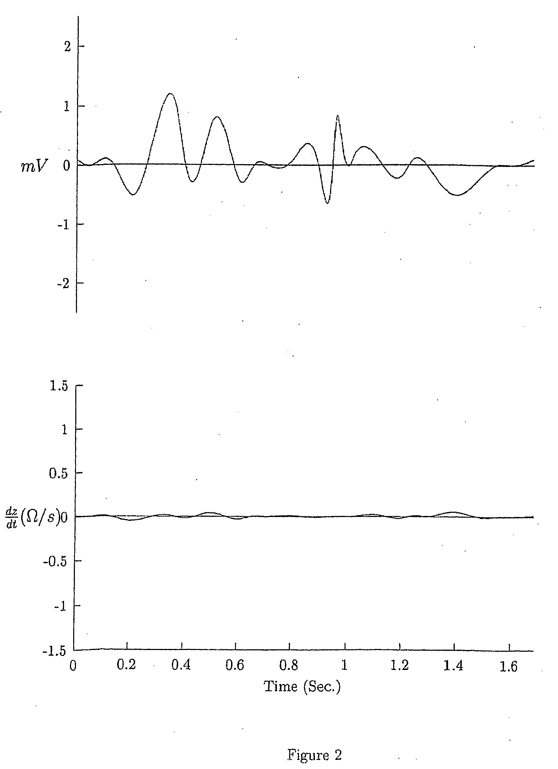

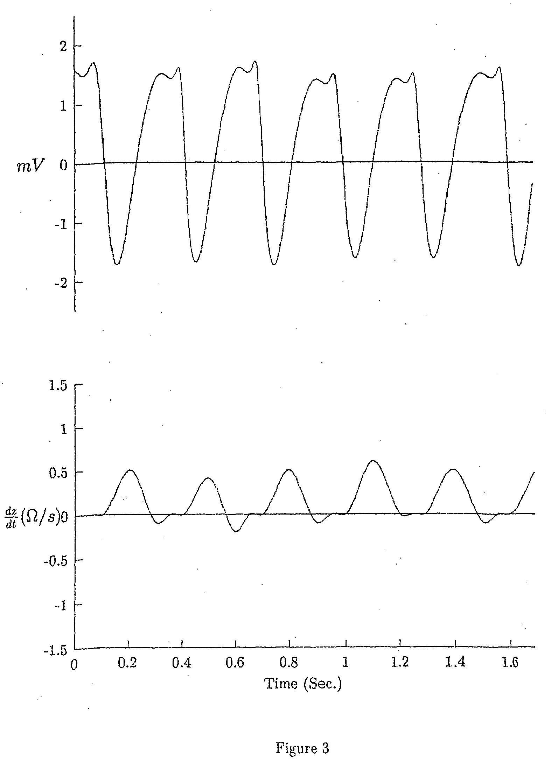

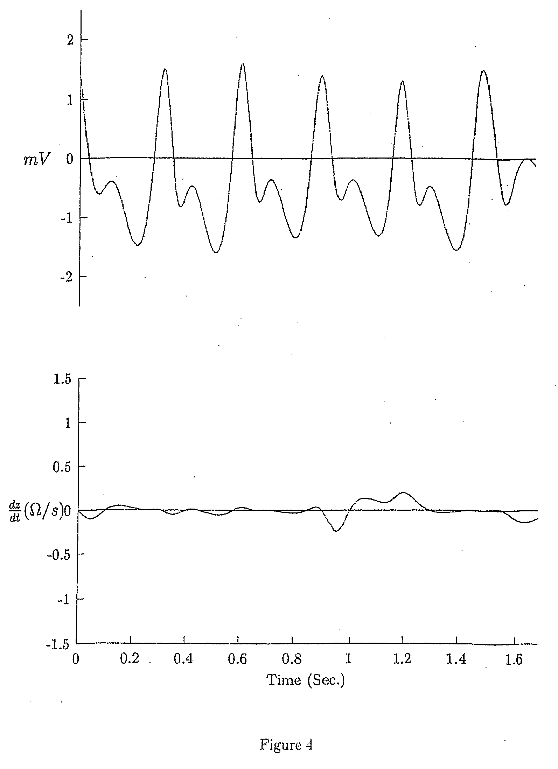

Note how the dz/dt (ICG) signal although very different in morphology, also possesses a beat by beat nature caused by the rhythmic pumping of the blood through the heart, thereby causing a change in the impedance of the thorax. Comparing this to Figure 2 we see that the amount of blood flowing during ventricular fibrillation (VF) is almost negligble. Although the difference between VF and SR is obvious, the ICG really becomes invaluable when we compare a shockable from a non-shockable ventricular tachycardia (VT). Figure 3 shows again the rhythmic pumping of blood flow taken from a patient suffering an acute VT. An un-sychronised shock is not advised in this case. Comparing this now to Figure 4 we again see the lack of blood flow. Failing to shock this patient will result in death because there is insufficient blood flow to the brain. Before we leave these figures however, note the ECG's at the top of both Figures 3 and 4. One can clearly see how difficult it is for any automated algorithm to distinguish between them. Of final note is that all of these traces were taken under controlled circumstances and while the ICG traces are clean and steady, these are not typical of what one will acquire when attempting to record an ICG from a patient in suspected cardiac arrest, in a public place by a first responder.

There are many scientific publications relating to the use of the ICG in measuring cardiac output. All of these publications however fail to address the one main problem still to be solved before the ICG can be used in a practical setting. There are various methods of acquiring an ICG signal. They all involve an alternating signal being applied across the sensing electrodes. This signal is either designed to possess a fixed voltage or a fixed current. The effect that the object under test exhibits on the test signal is measured and the impedance for the object is then calculated. A component of this measurement is the impedance of the blood within the heart. As this blood volume changes during a cardiac cycle, the measured impedance changes. Unfortunately the impedance measured due to blood volume change within the heart is considerably less than the total impedance of the subjects torso. This means that the ICG is sensed as a small change in value of a much larger baseline impedance. Other factors such as volume change due to inspiration and expiration also affect the measurement. Even very slight muscle activity and motion can affect the electrode contact impedance and considerably affect measurement. All these factors result in the use of the ICG for measurement of cardiac output being restricted to laboratory or controlled conditions.

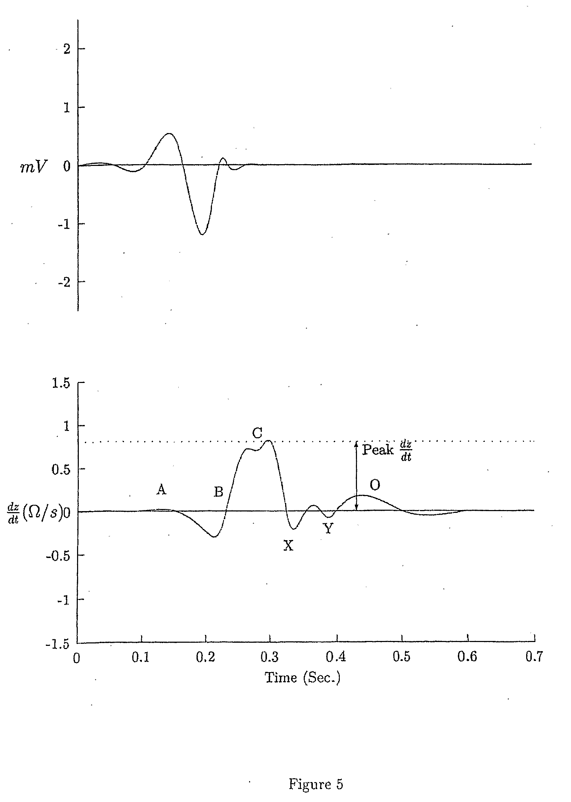

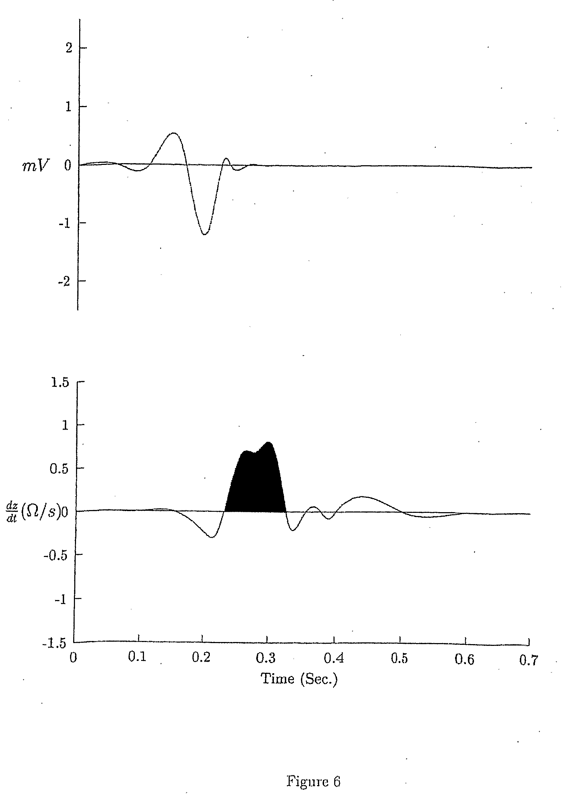

There are many techniques known to those skilled in the art which can reduce the effects of these factors. Some methods involve filtering, signal averaging[7], Fourier analysis and differential impedance measurement. There are also documented methods which analyse the ICG once it has been acquired. Typically these have involved the use of mathematical derivative and integrative processes attempting to glean measurement of cardiac output from the ICG. Figures 5 and 6 show the measurement of peak dz/dt and area under the C wave which are just two parameters reported to have a quantitative relationship to cardiac output. Figure 5 also shows how the various parts of the dz/dt waveform are labelled by convention[8]. Unfortunately these parameters and techniques have been somewhat unreliable. The baseline impedance, the structure of the heart and the subjects vascular demands all vary from one individual to the next. These previous attempts all share the limitation that they are determining to measure blood flow through the heart while employing a signal source that contains much interference due to variable factors that are not desirable.

A device according to the preamble of claim 1 is known from US2003109790.

According to an aspect of the present invention, there is provided a method of determining whether events in a heart's electrical activity result in corresponding blood flow events as specified in claim 1. According to another aspect of the present invention, there is provided an external defibrillator as specified in claim 5.

According to the present invention which is defined in claim 1, there is provided a method of determining whether events in a heart's electrical activity result in corresponding blood flow events, the method comprising:

> simultaneously taking an electrocardiograph (ECG) and an impedance cardiograph (ICG) of the heart;
> examining successive periods of the ECG to detect successive occurrences of at least one periodic waveform feature indicative of electrical activity of the heart;
> defining a period of time following each such detection;
> examining the ICG, or a signal derived therefrom, in each said search period to detect the occurrence of a waveform feature ("corresponding waveform feature") indicative of blood flow resulting from the detected occurrence in the ECG; and
> generating a signal indicative of the degree of concordance of the ICG with the ECG as a function of measurements made in respect of said detected waveform features.

The invention further provides an external defibrillator having circuitry arranged to implement the method specified above and to selectively advise a shock dependent upon the value of said signal indicative of the degree of blood flow.

An embodiment of the invention will now be described, by way of example, with reference to the accompanying drawings, in which:

> Figure 1 shows an example ECG signal from a subject in normal SR (top), together with corresponding time synchronised ICG signal (bottom).
> Figure 2 shows an example ECG signal from a subject in VF (top), together with corresponding time synchronised ICG signal (bottom).
> Figure 3 shows an example ECG signal from a subject in VT (top), together with corresponding time synchronised ICG signal (bottom) - this person requires alternate therapy and should not receive an unsychronised shock.
> Figure 4 shows an example ECG signal from a subject in VT (top), together with corresponding time synchronised ICG signal (bottom) - this person requires an emergency terminating shock.
> Figure 5 shows the measurement of the peak dz/dt parameter used in the determination of cardiac output.
> Figure 6 shows the measurement of the area under the C wave, another parameter used in the determination of cardiac output.

Figure 7 shows the result of the wave detection process for both R waves in the ECG and C waves in the dz/dt ICG signal.

Figure 8 shows an example comparison between the ECG and ICG.

Figure 9 shows the a block diagram of an embodiment of the invention.

As previously described the prior art has included many techniques that employ parameters determined from the ICG. The intent has been to measure the amount of cardiac output to subsequently determine whether or not to deliver therapy. Any practical and theoretical attempts to provide more accurate diagnosis by AED algorithm's have therefore used this determination in various ways to augment the determination made from the ECG.

However, the following presents a novel method which does not place the emphasis on measuring the amount of cardiac output or blood flow with any serious degree of accuracy. The basis of this may not immediately appear to be a directive that could possibly furnish a solution to the overall problem. It has however achieved exactly that.

The essence of the method involves "gating" the information supplied by the ICG with that obtained from the ECG. There is no direct attempt to quantitatively measure blood flow with any degree of accuracy. Rather the aim is to qualitatively determine whether or not there is any concordance between the cardiac events depicted by the ECG and those demonstrated by the ICG. This novel approach of ECG-ICG event gating has proved very effective in discriminating shockable from non-shockable VT's in both human and animal subjects, and it is likely that the approach is applicable to other conditions such as atrial flutter/fibrillation and VF. It must be appreciated that the gating can be applied to any rhythm and use a multitude of parameters measured from both ECG an ICG without departing from the scope of the invention.

The gating uses ECG and ICG signals which have first been processed using methods well know to those skilled in the art. In the case of the ICG these remove as much interference, contamination and variation as possible while leaving the fundamental ICG intact. Unlike the prior art however, this pre-processing can be performed much more aggressively. The reason for this is that the invention does not require an accurate quantitative measurement of the cardiac output. The pre-processing design has therefore a far larger degree of freedom to clean the signal, thereby almost totally removing interference due to movement, etc., a solution not previously possible using other approaches. The frequency bandwidth overlap of ECG content and interference has always been a troublesome dilemma for bio-medical engineers. The electrical activity of the heart occurs much faster than the mechanical contraction, which means that the ICG content due to heart contraction and cardiac output results in a content bandwidth in the ICG that incorporates more lower frequencies. This means that the dilemma is much worse for the ICG than that of the ECG. With many of the pre-processing constraints removed however the signal conditioning that can be used for this invention provides a signal that has a far higher signal to noise ratio than can be used by the prior art. This means the ultimate result is more reliable especially in "out of hospital" emergency response situations.

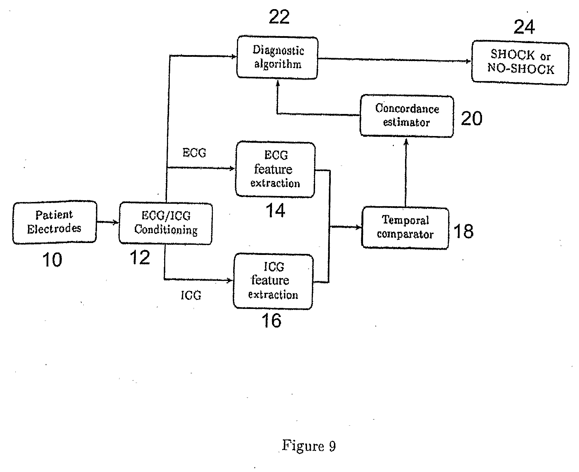

Figure 9 is a block diagram of an embodiment of the invention as implemented in an automatic external defibrillator (AED). Other than the patient electrodes 10, the conventional components of an AED are well-known, and are not shown in the drawing.

The AED comprises conventional patient electrodes 10 which are applied to the patient to obtain both the ECG and ICG waveform signals simultaneously. Signal conditioning circuitry 12 filters each signal to a bandwidth of 3-20Hz using analog filters with a Butterworth response. The circuitry 12 further differentiates the ICG to obtain dz/dt as the ICG signal for subsequent analysis, and individually but simultaneously digitises the ECG and differentiated ICG signals. The digitised ECG and ICG signals are passed to respective microprocessor-based feature extraction circuits 14, 16 respectively.

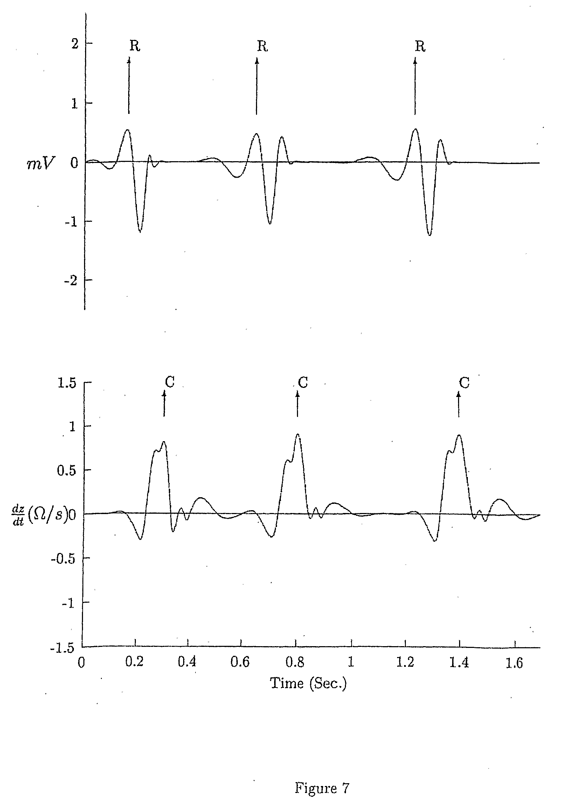

The circuit 14 examines successive heartbeat cycles (periods) of the ECG to detect successive occurrences of each of a plurality of periodic waveform features $E_1$ to $E_N$ indicative of electrical activity of the heart. One such feature is the R wave, shown at the top of Figure 7 for successive periods of the ECG. Other such features which may be detected, such as the Q wave, are well-known to those skilled in the art.

The circuit 16 examines successive heartbeat cycles (periods) of the ICG to detect successive occurrences of each of a plurality of periodic waveform features $I_1$ to $I_N$ indicative of blood flow in the heart. However, the ICG waveform features $I_1$ to $I_N$ are not arbitrarily chosen, but each is paired with one of the ECG waveform features $E_1$ to $E_N$ such that each pair $E_n$, $I_n$ (0<n<N+1) has an electromechanical relationship. By this it is meant that the particular ECG waveform feature $E_n$ results (in a healthy heart) in the corresponding ICG waveform feature $I_n$. For the the R wave in the ECG, the paired waveform feature in the ICG is the C wave, shown at the bottom of Figure 7 for successive periods of the ICG. To extract the R and C wave features a wave detector is separately used on both signals[9], the ECG detector being optimised for R wave detection and the ICG detector for C wave detection.

Next, the detected features are passed to a temporal comparator 18. This defines a period of time (search period) following the detection of each ECG waveform feature $E_n$ and examines the ICG waveform for the presence of the paired feature In during that search period. The duration of the search period is calculated from heart-rate dependant measurements made on the ECG and is designed such that the paired feature (if present) should appear within that time period following the detected ECG feature. The search period duration will therfore depend on the heart rate and the particular waveform features selected. In this particular embodiment the search period was calculated as twice the period of the QRS interval in the ECG.

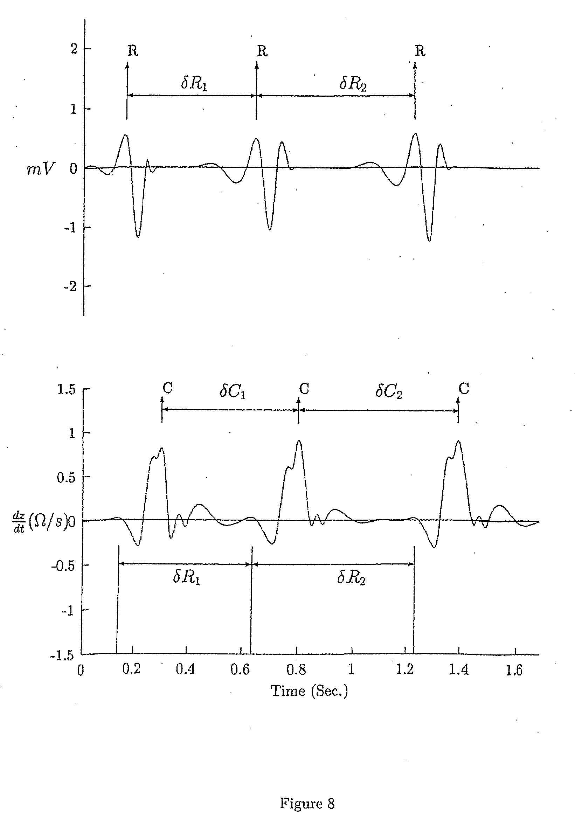

Where a valid pair is detected, i.e. where the ICG feature In is found within the search period following the related ECG feature $E_n$, a measurement (hereinafter referred to as a parameter) is made in respect of each feature of the pair $E_n$, $I_n$. The parameters are not chosen arbitrarily, but are equivalent for the feature $E_n$ and the feature In. Thus if the parameter for $E_n$ is the width of the R wave, the equivalent parameter for $I_n$ is the width of the C wave. Other parameter pairs are the height of R wave in the ECG and the height of C wave in the ICG, the ratio of the areas under the R and Q waves in the ECG and the ratio of the areas under the C and X waves in the ICG, and other parameter pairs which would be known to those skilled in the art. In the example shown in Figure 8, the parameter measured in respect of each R wave is the R-R interval $\delta R_1$, $\delta R_2$, etc. to the next R wave, and the equivalent parameter measured in respect of each C wave is the C-C interval $\delta C_1$, $\delta C_2$, etc.

This process, applied to all the features $E_1$ to $E_N$ in each period of the ECG waveform, generates for each waveform period a respective set of parameter pairs $X_1^{src}$ to $X_N^{src}$ where *src* denotes the source of the parameter ECG or ICG. Thus $X_1^{ECG}$ represents the value of parameter number one for the ECG and $X_1^{ICG}$ the value of its ICG pair. For the case where a paired feature was not found, and hence a corresponding ICG parameter could not be measured, a large error value was substituted.

Next, the parameter pairs $X_1^{src}$ to $X_N^{src}$ are passed to a concordance estimator 20. In this embodiment the estimator generates the linear sum of all the comparisons of all the parameter pairs:-

$$Y = \sum_{n=0}^{N} X_n^{ECG} - X_n^{ICG} \qquad (2)$$

For the case particular parameters shown in Figure 8, the difference between the pair is:

$$\partial X_1 = \left\lfloor X_n^{ECG} \right\rfloor_{R-R} - \left\lfloor X_n^{ICG} \right\rfloor_{C-C} = \partial R_1 - \partial C \qquad (3)$$

where $X_1$ is the identifier for that parameter pair.

Since in this particular example the parameter pairs are chosen such that they should have very little difference during concordance, this means that the estimate Y furnished a very low value when there was concordance between the ECG and ICG and a very large value when there was bad correlation or during dissassociation when a large error value contributed. Thus it will be recognised that *Y* is indicative of the lack of concordance of the ICG with the ECG, the larger Y the higher the lack of concordance (it will be appreciated that a high degree of concordance indicates a rythmic blood flow which is life-supporting and the patient does not need a shock).

Finally the estimate Y was selectively used by a diagnostic algorithm 22 which ignored its value for various rhythms but compared its value to a predetermined threshold when it had already classified the rhythm as VT in addition to several other criteria outside the scope of this specification. If the concordance estimate was below the threshold for the given VT then no shock was advised. Therefore, as a final qualification a shock was advised if:

$$Y \geq E_{th} \qquad (4)$$

Thus the shock circuitry 24 of the AED gave a shock or no shock advisory according to the value of Y.

This technique proves very effective in reducing false positives for the algorithm 22. The result was also consistent when signals were analsed which contained a large degree of impedance interference. The use of the technique is thought to be useful for application to rhythms other than VT, and it must be appreciated that this will not depart from the scope of the invention.

The technique described above provides a novel qualitative approach applied after the ICG has been acquired and conditioned. This trechnique overcomes the problems described in relation to the prior art and provides a method whereby the ICG can be employed in a practical environment to reliably discern shockable from non-shockable cardiac rhythms.

The invention is not limited to the embodiment described herein which may be modified or varied without departing from the scope of the invention.

References

1. Pantridge J.F. et al. A Mobile Intensive-Care Unit in the Management of Myocardial Infarction. Lance (1967) ii, 271-273.

2. Anderson J.McC. *et al.* Apparatus for Pattern Recognition of ECG Signals for Detecting Fibrillation. U.S. Parent (1984) 4,453,551.

3. American Heart Association - International Consensus on Science The Automatic External Defibrillator. Guidelines 2000 for Cardiopulmonary Resuscitation and Emergency Cardiovascular Care, Circulation (2000) 1 02(Supp 1,) 60- 76.

4. Ornato J.P. et al. Inappropriate Electrical Counter-shocks by an Automated External Defibrillator. Annals of Emergency Medicine (1992) 21, 1278-1282..

5. Sedgwick M.L et al. Efficacy of Out of Hospital Defibrillation by Ambulance Technicians Using Automated External Defibrillators. Resuscitation (1992) 24, 73-87.

6. Cummins R.O. et al. Ventricular Fibrillation. Automatic External Defibrillators and the United States Food and Drug Administration Confrontation Without Comprehension. Annals of Emergency Medicine (1995) 26, 621-631.

7. Johnston P.W. et al. The Transthoracic Impedance Cardiogram is a Potential Haemodynamic Sensor for an Automated External Defibrillator. European Heart Journal (1998) 19, 1879-1888.

8. Woltjer H.H. et al. The Technique of Impedance Cardiography. European Heart Journal (1997) 18, 1396-1.403.

9. Pan J. et al. A Real-time QRS Detection Algorithm. IEEE Transactions on Biomedical Engineering (1985) BME-32 (3), 230-236.

**Claims**

1. A method of determining whether events in a heart's electrical activity result in corresponding blood flow events, the method comprising the following steps:

   (a) simultaneously taking an electrocardiograph (ECG) and an impedance cardiograph (ICG) of the heart;
   (b) examining successive heartbeat cycles of the ECG to detect successive occurrences of a plurality of different periodic waveform features each indicative of electrical activity of the heart;
   (c) defining a period of time ("search period") following each such detection;
   (d) examining the ICG in each said search period to detect the occurrence of a waveform feature in the ICG corresponding to the detected ECG waveform feature and indicative of blood flow resulting from the detected ECG waveform feature; and
   **characterised by**
   in respect of each heartbeat cycle, generating a signal indicative of the degree of concordance of the ICG with the ECG by:
   (e) for each pair of corresponding features detected in step (d), measuring equivalent parameters in respect of each feature of the pair; and
   (f) summing the difference between the measured parameters of each pair of corresponding features.

2. A method as claimed in claim 1, wherein one of the different periodic waveform features in the ECG is the R wave and the corresponding waveform feature in the ICG is the C wave.

3. A method as claimed in claim 2, wherein the R-R interval is measured in respect of the detected ECG waveform feature and the C-C interval is measured in respect of the detected ICG waveform feature.

4. A method as claimed in any preceeding claim, wherein the search period is calculated from a heart-rate dependent measurement made on the ECG.

5. An external defibrillator having circuitry arranged to implement the method claimed in any preceding claim and to selectively advise a shock dependent upon the degree of concordance.

**Patentansprüche**

1. Verfahren zur Bestimmung, ob Ereignisse der elektrischen Aktivität eines Herzens entsprechende Blutflussereignisse zur Folge haben, wobei das Verfahren folgende Schritte umfasst:

(a) gleichzeitiges Erstellen eines Elektrokardiogramms (EKG) und eines Impedanzkardiogramms (IKG) des Herzens;
(b) Prüfen von aufeinanderfolgenden Herzschlagzyklen des EKGs, um ein aufeinanderfolgendes Auftreten einer Vielzahl von unterschiedlichen periodischen Wellenformmerkmalen zu erfassen, die jeweils auf eine elektrische Aktivität des Herzens hinweisen;
(c) Definieren eines Zeitraums ("Suchzeitraum") nach jeder derartigen Erfassung;
(d) Prüfen des IKGs in jedem genannten Suchzeitraum, um das Auftreten eines Wellenformmerkmals in dem IKG zu erfassen, das dem erfassten EKG-Wellenformmerkmal entspricht und auf einen Blutfluss hinweist, der aus dem erfassten EKG-Wellenformmerkmal resultiert; und
**gekennzeichnet durch**
Erzeugen eines Signals in Bezug auf jeden Herzschlagzyklus, das den Grad der Übereinstimmung des IKGs mit dem EKG anzeigt, **durch** folgende Maßnahmen:
(e) für jedes Paar von entsprechenden Merkmalen, die in Schritt (d) erfasst wurden, Messen von äquivalenten Parametern in Bezug auf jedes Merkmal des Paares; und
(f) Addition der Differenz zwischen den gemessenen Parametern von jedem Paar mit entsprechenden Merkmalen.

2. Verfahren nach Anspruch 1, wobei eines der unterschiedlichen periodischen Wellenformmerkmale in dem EKG die R-Welle ist und das entsprechende Wellenformmerkmal in dem IKG die C-Welle ist.

3. Verfahren nach Anspruch 2, wobei das R-R-Intervall in Bezug auf das erfasste EKG-Wellenformmerkmal gemessen wird und das C-C-Intervall in Bezug auf das erfasste IKG-Wellenformmerkmal gemessen wird.

4. Verfahren nach einem der vorhergehenden Ansprüche, wobei der Suchzeitraum ausgehend von einer herzfrequenzabhängigen Messung berechnet wird, die an dem EKG vorgenommen wurde.

5. Externer Defibrillator, der eine Schaltung aufweist, die für Folgendes angeordnet ist: Implementieren des Verfahrens nach einem der vorhergehenden Ansprüche und selektives Empfehlen eines Schocks abhängig von dem Grad der Übereinstimmung.

**Revendications**

1. Procédé servant à déterminer si des événements au niveau de l'activité électrique cardiaque produisent des événements de circulation sanguine correspondants, le procédé comportant les étapes suivantes consistant à :

(a) effectuer simultanément un électrocardiogramme (ECG) et un électrocardiogramme par impédance (ICG) du coeur ;
(b) examiner les cycles successifs de battements du coeur de l'ECG afin de détecter des apparitions successives d'une pluralité de différentes caractéristiques de formes d'ondes périodiques indiquant chacune l'activité électrique du cour ;
(c) définir une période de temps (« période de recherche ») à la suite de chaque détection de ce type ;
(d) examiner l'ICG au cours de chaque dite période de recherche afin de détecter l'apparition d'une caractéristique de forme d'onde dans l'ICG qui correspond à la caractéristique de forme d'onde détectée de l'ECG et indiquant la circulation sanguine résultant de la caractéristique de forme d'onde d'ECG détectée ; et
**caractérisé par**, par rapport à chaque cycle de battements de coeur, l'étape consistant à générer un signal indiquant le degré de concordance entre l'ICG et l'ECG en effectuant les étapes consistant à :
(e) pour chaque paire de caractéristiques correspondantes détectées au cours de l'étape (d), mesurer des paramètres équivalents par rapport à chaque caractéristique de la paire ; et
(f) additionner la différence entre les paramètres mesurés de chaque paire de caractéristiques correspondantes.

2. Procédé selon la revendication 1, dans lequel l'une des différentes caractéristiques de formes d'ondes périodiques dans l'ECG est l'onde R et la caractéristique de forme d'onde correspondante dans l'ICG est l'onde C.

**3.** Procédé selon la revendication 2, dans lequel l'intervalle R-R est mesuré par rapport à la caractéristique de forme d'onde détectée de l'ECG et l'intervalle C-C est mesuré par rapport à la caractéristique de forme d'onde détectée de l'ICG.

**4.** Procédé selon l'une quelconque des revendications précédentes, dans lequel la période de recherche est calculée d'après une mesure qui est fonction du rythme cardiaque et qui est effectuée sur l'ECG.

**5.** Défibrillateur externe ayant des circuits agencés à des fins de mise en oeuvre du procédé selon l'une quelconque des revendications précédentes et permettant de conseiller de manière sélective une décharge en fonction du degré de concordance.

Figure 1

Figure 2

Figure 3

Figure 4

Figure 5

13

Figure 6

Figure 7

Figure 8

Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2003109790 A

- US 4453551 A

### Non-patent literature cited in the description

- **PANTRIDGE J.F. et al.** A Mobile Intensive-Care Unit in the Management of Myocardial Infarction. *Lance,* 1967, vol. ii, 271-273
- International Consensus on Science The Automatic External Defibrillator. Guidelines 2000 for Cardiopulmonary Resuscitation and Emergency Cardiovascular Care, Circulation. American Heart Association, 2000, vol. 1 02, 60-76
- **ORNATO J.P et al.** Inappropriate Electrical Counter-shocks by an Automated External Defibrillator. *Annals of Emergency Medicine,* 1992, vol. 21, 1278-1282
- **SEDGWICK M.L et al.** Efficacy of Out of Hospital Defibrillation by Ambulance Technicians Using Automated External Defibrillators. *Resuscitation,* 1992, vol. 24, 73-87

- **CUMMINS R.O. et al.** Ventricular Fibrillation. Automatic External Defibrillators and the United States Food and Drug Administration Confrontation Without Comprehension. *Annals of Emergency Medicine,* 1995, vol. 26, 621-631
- **JOHNSTON P.W. et al.** The Transthoracic Impedance Cardiogram is a Potential Haemodynamic Sensor for an Automated External Defibrillator. *European Heart Journal,* 1998, vol. 19, 1879-1888
- **WOLTJER H.H. et al.** The Technique of Impedance Cardiography. *European Heart Journal,* 1997, vol. 18, 1396-1.403
- **PAN J. et al.** A Real-time QRS Detection Algorithm. *IEEE Transactions on Biomedical Engineering,* 1985, (3), 230-236